# EUROPÄISCHE PATENTSCHRIFT

(11) **EP 0 937 105 B1**
(45) Veröffentlichungstag und Bekanntmachung des Hinweises auf die Patenterteilung: **01.10.2003**
(21) Anmeldenummer: 97931659.3
(22) Anmeldetag: 25.06.1997
(51) Int. Cl.: C07K 16/42, A61K 39/00

(54) **DNA, kodierend für die variable Region eines anti-idiotypischen Antikörpers**
DNA encoding the variable region of an anti-idiotypic antibody
ADN codant pour la région variable d'un anticorps anti-idiotypique

(30) Priorität: 27.06.1996 DE 19627352
(43) Veröffentlichungstag der Anmeldung: 25.08.1999
(73) Patentinhaber: MAX-DELBRÜCK-CENTRUM FÜR MOLEKULARE MEDIZIN, 13125 Berlin (DE)
(72) Erfinder: KARSTEN, Uwe, D-10407 Berlin (DE)
(74) Vertreter: Baumbach, Friedrich
(86) Internationale Anmeldenummer: DE9701327
(87) Internationale Veröffentlichungsnummer: WO98000444

(56) Entgegenhaltungen:
- WO-A-95/04548
- GRZYCH J. M. ET AL.,: "An anti-idiotype vaccine against experimental schistosomiasis" NATURE, Bd. 316, - 4.Juli 1985 Seiten 74-76, XP002044767
- ULMER J B ET AL: "HETEROLOGOUS PROTECTION AGAINST INFLUENZA BY INJECTION OF DNA ENCODING A VIRAL PROTEIN" SCIENCE, Bd. 259, 19.März 1993, Seiten 1745-1749, XP002009751
- KLEIN R. L. ET AL.,: "T-Cryptantigen exposure in neonatal necrotizing enterocolitis" J. PEDIATRIC SURGERY, Bd. 21, Nr. 12, - 1986 Seiten 1155-1158, XP002045264

## Beschreibung

Die Erfindung betrifft eine Vakzine gegen Kohlenhydrat-Antigene, Verfahren zu ihrer Herstellung und ihre Verwendung.

Zur Immunisierung mit einem herkömmlichen Antigen gibt es derzeit zwei Alternativen, die Immunisierung mit einer Desoxyribonukleinsäure (DNA) oder die Immunisierung mit einem anti-idiotypischen Antikörper.

Die erstere ist bisher nur auf Peptid- bzw. Protein-Antigene anwendbar. Von einem solchen Antigen bzw. von dessen immunogenem Epitop wird eine cDNA hergestellt, die dann intramuskulär (i.m.) oder intradermal (i.d.) injiziert wird. Auf diese Weise kann eine humorale und zelluläre Immunantwort mit sehr effektiver protektiver Wirkung erreicht werden (Wolff, J.A., et al., Science 247: 1465, 1990; Ulmer, J.B., et al., Vaccine 12: 1541, 1994; Raz, E., et al., Cancer Res. 52: 1954, 1992).

Anti-idiotypische Antikörper sind bekanntermaBen gegen die Erkennungsstruktur (variable Domäne) eines Antikörpers (Ab1) gerichtete sekundäre Antikörper (Ab2). Solche Antikörper werden im Körper in geringer Menge ständig im Rahmen des immunologischen Regelsystems gebildet (Netzwerktheorie; Jerne, N.K., Ann.Immunol.(Paris) 125C: 373, 1974). Ab2 können in ihrer Erkennungsstruktur ein Abbild ("internal image") des Antigens, das zur Entstehung von Ab1 geführt hat, sein. Ist dies der Fall, so kann mit einem solchen anti-idiotypischen Antikörper (Ab2, Typ β) in gleicher Weise und mit dem gleichen Ergebnis immunisiert werden wie mit dem Antigen; es werden also Ab1 und gegebenfalls zytotoxische Zellen gegen das ursprüngliche Antigen induziert.
Der Ansatz einer Immuntherapie bei Krebserkrankungen geht davon aus, daß es möglich ist, die natürliche Immunabwehr zu verstärken. Der typische Anwendungsfall ist die Bekämpfung der Residualerkrankung (Metastasenprophylaxe) nach einer konventionellen Therapie (z.B. chirurgischer Entfernung der Hauptmenge der Tumorzellen).

Tumoren unterscheiden sich von den entsprechenden normalen Körperzellen nicht nur im biologischen Verhalten, sondern auch biochemisch. Tumorassoziierte Antigene lösen im Rahmen der Immunüberwachung ("Immunosurveillance") im Körper Abwehrreaktionen aus. Diese reichen jedoch nicht in allen Fällen aus, das Auswachsen eines Tumor zu verhindern. Vielfach üben sich entwickelnde Tumoren eine immunsuppressive Wirkung aus, wobei die zugrundeliegenden Mechanismen verschieden sein können.

Sehr häufig sind tumorassoziierte Antigene Kohlenhydrate (CH-Ag, Hakomori, S.-I., Curr.Opinion Immunol. 3: 646, 1991).

CH-Antigene bewirken in der Regel eine starke humorale Immunantwort, die aber normalerweise bei der Bekämpfung von Tumorzellen wirkungslos ist. Nur für wenige CH-Ag ist sicher gezeigt worden, daß sie auch eine zelluläre Immunantwort auslösen können. Es ist weitgehend unbekannt, ob und wie CH-Ag immunologisch präsentiert werden. Daher ist ihre Eignung für eine Tumor-Vakzine umstritten. Weiterhin ist die Synthese von Oligosacchariden weitaus aufwendiger als die von Peptiden oder von DNS.

Der Erfindung lag deshalb die Aufgabe zugrunde, ein DNA-Konstrukt für die Verwendung in einer Vakzine gegen Kohlenhydrat-Antigene bereitzustellen, die sich zur aktiven spezifischen Immunisierung von Erkrankungen eignet, die auf solche Antigene zurückzuführen sind, und die ein Ausbleiben einer zellulären Immunantwort vermeidet. Insbesondere soll die Vakzine zur spezifischen Immunisierung von Tumorerkrankungen geeignet sein.

Diese Aufgabe wird überraschend durch ein DNA-Konstrukt gelöst, das durch eine DNA, welche die variable Region eines monoklonalen anti-idiotypischen Antikörpers (Ab2) gegen einen monoklonalen anti-Kohlenhydrat-Antikörper (Ab1) kodiert, gekennzeichnet ist.

Als variable Region werden die terminalen Abschnitte der schweren und leichten Ketten von Immunglobulinen, die die Erkennungsstrukturen enthalten, verstanden.

Erfindungsgemäß wird das DNA-Konstrukt hergestellt, in dem man
- monoklonale Antikörper (Ab1) gegen Kohlenhydrat-Antigene und nachfolgend
- anti-idiotypische Antikörper (Ab2) vom Typ β, die die Kohlenhydrat-Antigene immunologisch imitieren, nach an sich bekannten Verfahren gewinnt und
- von diesen Ab2 oder von geeigneten Peptiden dieser Ab2 nach an sich üblichen Methoden eine entsprechende cDNA erzeugt.

Dabei können verschiedene Strategien verfolgt werden; z.B. cDNAs des variablen Teils der leichten und schweren Ketten des Ab2 im Gemisch oder allein oder geeignete Konstrukte, die Anteile beider Ketten enthalten.

Die Verwendung einer auf dem erfindungsgemäßen Konstrukt berückenden Vakzine ist besonders bei Tumorerkrankungen, insbesondere Residualerkrankungen von Patienten mit malignen Erkrankungen, anwendbar, bei denen Kohlenhydrate als tumorassoziierte Antigene eine Rolle spielen, wie zum Beispiel das Thomsen-Friedenreich-Antigen (TF) mit der Epitopstruktur Galβ1-3GalNAcl, das beim Menschen als "Pan-Karzinom-Antigen" auftritt (Springer, G.F., Science 224: 1198, 1984; Cao,Y., et al., Cancer 76: 1700, 1995).
Die vakzine ist aber auch zur Immunisierung gegen Infektionserreger anwendbar, bei denen das Zielantigen ein Kohlenhydrat ist.

Vorzugsweise erfolgt die Anwendung der Vakzine intramuskulär (i.m.) oder intradermal (i.d.).

Die Vakzine besitzt den Vorteil, daß die Vorzüge der DNA-Immunisierung erstmals auch gegen Kohlenhydrat-Antigene genutzt werden können. Man erhält eine humorale und zelluläre Immunantwort mit ausgesprochen effektiver protektiver Wirkung.

Die Problematik der immunologischen Präsentation von Kohlenhydrat-Antigenen und das mögliche Ausbleiben einer zellulären Immunantwort kann damit umgangen werden.

Anschließend wird die Erfindung durch Ausführungsbeispiele näher erläutert, auf die sie jedoch nicht beschränkt werden soll.

### Ausführungsbeispiele

### Beispiel 1:

### Herstellung der Hybridomzellinie A78-G/A7 und von Antikörpern

Inzuchtmäuse des Stammes Balb/c wurden intraperitoneal mit 100 mg Asialo-Glykophorin (Sigma, Deisenhofen) in PBS, gemischt mit komplettem Freundschem Adjuvans, immunisiert. Nach 24 h wurden 100 mg/kg Körpergewicht Cyclophosphamid in PBS, ebenfalls i.p. verabreicht. Zwei Wochen später (4 Tage vor Entnahme der Milzzellen) wurde mit 100 mg Asialo-Glykophorin ohne Adjuvans nachimmunisiert.
Aseptisch präparierte Milzzellen einer wie vorstehend beschrieben immunisierten Maus wurden nach Standardmethoden (Peters, H.H., et al.: "Monoklonale Antikörper, Herstellung und Charakterisierung", Springer, Berlin 1985; Karsten, U., et al., Methods in Enzymology 220, 228, 1993) mit Hilfe von Polyethylenglykol mit Maus-Myelomzellen der Zellinie X63-Ag8.653 (Kearney, J.F., et al., J.Immunol. 123, 1548, 1979) fusioniert.

Die Selektion der Hybridome erfolgte in Mikrotestplatten in einem Selektionsmedium mit Azaserin; das Hybridomwachstum wurde mit Peritoneal-Feederzellen von Balb/c-Mäusen gefördert.

Die Testung der Kulturüberstände auf spezifische Antikörper gegen das TF-Antigen erfolgte in Enzymimmunoassays sowie in der Immunfluoreszenz. Für den *Enzymimmunoassay* wurden Asialo-Glykophorin (2 mg/ml, 50 ml pro Well) bzw. Glykophorin (als Negativkontrolle; gleiche Konzentration) an die feste Phase von Mikrotestplatten gebunden. Dann wurden je 50 ml des zu prüfenden Kulturüberstandes dazugegeben und inkubiert. Der Nachweis spezifischer Antikörper erfolgte über Peroxidasemarkiertes Kaninchen-Anti-Maus-Ig nach üblichen Verfahren. Die Auswertung erfolgte mit einem Testplatten-Photometer (Spektra, SLT Labinstruments, Salzburg). Dabei wurde insbesondere nach Hybridom-Überständen gesucht, die mit Asialo-Glykophorin, nicht aber mit Glykophorin reagierten. Zwei Hybridome (A78-G/A7 und A78-H/C8) erfüllten diese Bedingung. Für den *Immunfluoreszenztest* wurden Zellen der Mammakarzinom-Zellinie T-47D (Keydar, I., et al., Eur.J.Cancer 15, 659, 1979) auf Multitest-objektträger als Monolayer-Kultur für 2 Tage aufwachsen gelassen, und zwar in zwei Sublinien (TF⁺ und TF⁻). Danach wurde das Kulturmedium abgesaugt, und die Präparate wurden trocknen gelassen. Der Test selbst wurde in üblicher Weise durch Inkubation mit Hybridom-Kulturüberständen und nachfolgend mit FITC-markiertem Ziegen-Anti-Maus-Ig durchgeführt und unter dem Fluoreszenzmikroskop (Jenamed fluorescence, C.Zeiss, Jena) ausgewertet. Im Immunfluoreszenztest wurden alle Kulturüberstände geprüft, die im Enzymimmunoassay positiv waren. Dabei wurde insbesondere nach solchen Überständen gesucht, die mit der TF⁺-Variante, nicht aber mit der TF⁻-Variante reagierten.

Darüber hinaus wurde die zytologische Lokalisation des Antigens registriert und mit der Reaktion von FITC-markiertem Erdnußlektin (PNA; Serva, Heidelberg) verglichen. Von den beiden im Enzymimmunoassay gefundenen Hybridora-Überständen erfüllte einer (A78-G/A7) die Bedingungen des Immunfluoreszenztests.
Das Hybridom A78-G/A7 wurde nach üblichen Methoden ( Peters, H.H., et al.,l.c.) durch limitierende Verdünnung mehrfach kloniert und für die Langzeitlagerung bei -196 °C eingefroren (Karsten, U., in Friemel, H.,Hrsg., "Immunologische Arbeitsmethoden", 4.Aufl., S.311ff., Fischer, Jena, 1991).

Die erfindungsgemäße Produktion des Antikörpers der Hybridomzellinie A78-G/A7 erfolgte in üblichen Flaschenkulturen. Kulturüberstand aus diesen Kulturen wurde in allen folgenden Spezifitätstests und immunzytologischen sowie immun-histologischen Untersuchungen eingesetzt (siehe Beispiele 3-5).

Isotypenbestimmung: Der Isotyp des Antikörpers A78-G/A7 wurde mit einem kommerziellen Testkit (Pharmingen, San Diego, USA) als IgM, kappa ermittelt.

### Beispiel 2 :

### Herstellung der Hybridomzellinie A68-B/A11 und von Antikörpern

Inzuchtmäuse des Stammes Balb/c wurden intraperitoneal mit einer Mischung von TFB-BSA (synthetischem TFB, über O-CETE an BSA gekoppelt; Janssen Biochemica, Beerse, Belgien) in PBS und komplettem Freundschem Adjuvans (Difco Laboratories, Detroit, USA) immunisiert. In Abständen von > 1 Mo. wurde dreimal ohne Zusatz von Adjuvans nachimmunisiert. Vier Tage nach der letzten Immunisierung wurden Milzzellen einer dieser Mäuse nach Standardmethoden (Peters, H.H., et al., "Monoklonale Antikörper, Herstellung und Charakterisierung", Springer, Berlin, 1985) mittels Polyethylenglykol mit Maus-Myelomzellen der Zellinie X63-Ag8.653 (Kearney, J.F., et al., J.Immunol. 123, 1548, 1979) fusioniert. Die Selektion der Hybridome erfolgte mittels Azaserin in Mikrotestplatten; das Wachstum wurde durch Peritoneal-Feederzellen unterstützt.

Das Screening der Hybridom-Kulturüberstände auf spezifische Anti-TF-Antikörper wurde mittels *Enzymimmunoassays* und *Immunfluoreszenztests* durchgeführt. Im ersten Fall dienten TFB-BSA und BSA (Negativkontrolle), an Mikrotestplatten gebunden, als Antigene. Diese wurden mit 50 ml Hybridom-Kulturüberstand pro Well inkubiert. Der Nachweis spezifisch gebundener Antikörper wurde in üblicher Weise mit Peroxidase-konjugiertem Zweitantikörper vorgenommen. *Immunfluoreszenztests* wurden an Multitest-Objektträgern ausgeführt, die mit einer Zell-Monolayer der Mammakarzinom-Zellinie MCF-7 (Soule, H.D., et al., J.Nat.Cancer Inst. 51, 1409, 1973) bewachsen waren. Zum Nachweis spezifischer Hybridom-Antikörper diente in diesem Fall FITC-markiertes Antiserum gegen Maus-Ig.

Bei der Untersuchung der aus dem Experiment hervorgegangenen Hybridome wurden mehrere gefunden, die im Enzymimmunoassay mit TFB-BSA, nicht aber mit BSA reagierten (A68-B/All, A68-E/A2, A68-E/E3). Unter diesen Antikörpern zeigte jedoch nur A68-B/A11 eine Reaktion mit MCF-7-Zellen.
Das Hybridom A68-B/A11 wurde nach der Methode der limitierenden Verdünnung mehrmals kloniert, zur Langzeitlagerung bei -196 °C eingefroren und erfindungsgemäß zur Antikörperproduktion durch Kultivierung oder nach Transplantation herangezogen.

Als Isotyp des Antikörpers A68-B/A11 wurde IgM, kappa ermittelt.

### Beispiel 3:

### Reaktionsspektrum der monoklonalen Antikörper A78-G/A7 und A68-B/All

### 3.1 Reaktionsspektrum in Enzymimmunoassays mit einer Anzahl von Antigenen

Als Methode dienten übliche Enzymimmunoassays in Mikrotestplatten mit an die feste Phase adsorbierten Antigenen.

Nach Inkubation mit dem monoklonalen Antikörper in verschiedenen Verdünnungen erfolgte der Nachweis mit Peroxidase-markiertem Anti-Maus-Ig und anschließender Farbreaktion mit o-Phenylendiamin. Die Extinktionen wurden mit einem Testplattenphotometer (Spectra, SLT Labinstruments) gemessen und ausgewertet (Tab.1).

**Tabelle 1:**

| Reaktion der monoklonalen Antikörper A78-G/A7 und A68-B/A11 (Spezifität: | | | |
|---|---|---|---|
| Thomsen-Friedenreich-Antigen) mit TF⁺ und TF⁻ Antigenen | | | |
| Antigen | TF | A78-G/A7 | A68-B/A11 |
| Glykophorin | - | - | - |
| Asialo-Glykophorin | + | ++ | (+) |
| TFₐ-BSA | + | (+) | ++ |
| TF_{β}-BSA | + | + | +++ |
| BSA | - | - | - |
| Anti-freeze glycoprotein | + | + | (+) |
| Asialo-Mucin aus Rindersperma | + | +++ | + |
| Gangliosid G_{M1} | - | - | - |
| Asialo-G_{M1} | + | + | + |
| Asialo-Fetuin | - | - | - |

### 3.2 Immunabsorptionsprofile der monoklonalen Antikörper A78-G/A7 und A68-B/A11

Methode: Antikörperhaltige Kulturüberstände wurden mit trägergebundenen synthetischen Oligosacchariden (Synsorb, Chembiomed, Edmonton, Canada) inkubiert und ihr Titer vor und nach Immunabsorption verglichen.

**Tabelle 2:**

| Absorptionsprofile mit synthetischen Immunosorbentien (Synsorb). Symbole: + = vollständige, (+) = partielle, - = keine oder marginale Absorption. | | |
|---|---|---|
| Synsorb-Typ (Antigen) | A78-G/A7 | A68-B/A11 |
| T (GalB1-3GalNAc-) | + | + |
| A (Blutgruppe A) | (+) | + |
| Le^{c} (Galβ1-3GlcNAc-) | + | - |
| Forssman (F.-Antigen) | - | + |
| Le^{a} (Lewis a) | - | - |
| Le^{b} (Lewis b) | - | - |
| Le^{x} (Lewis x) | c | - |
| N-Acetyllaktosamin | (+) | - |

### Beispiel 4:

### Charakteristik des Antikörpers A78-G/A7 (Anti-TF-Antigen)

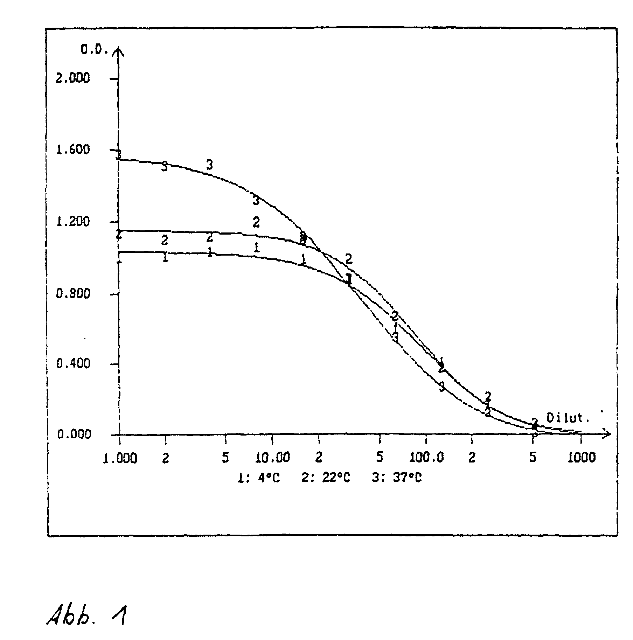

### 4.1 Temperaturabhängigkeit der Antigen-Antikörperbindung (Abb. 1)

Antigen: Asialo-Glykophorin, 2 mg/ml, 50 ml/Well. Abszisse:
Verdünnung des Antikörpers; Anfangskonzentration 45 mg/ml.

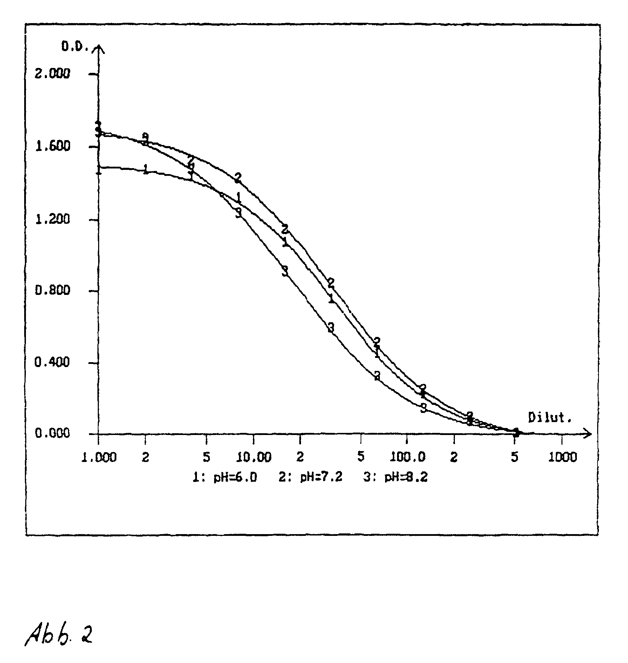

### 4.2 pH-Abhängigkeit der Antigen-Antikörperbindung (Abb.2).

Antigen: Asialo-Glykophorin, 2 mg/ml, 50 ml/Well. Abszisse:
Verdünnung des Antikörpers; Anfangskonzentration 45 mg/ml.

### Beispiel 5:

### Immunhistologischer Nachweis von Tumoren mit dem Antikörper A78-G/A7

### 5.1 Reaktion von menschlichen Zellinien mit A78-G/A7 (Tab.3).

Methodik: Immunfluoreszenztest an Zellen auf Multitestobjektträgern

**Tabelle 3:**

| Immunzytologie mit dem Antikörper A78-G/A7 (Spezifität: Thomsen-Friedenreich-Antigen) | | |
|---|---|---|
| Zellinie | Herkunftsgewebe | Reaktion |
| T-47D | Mammakarzinom | +/- |
| MCF-7 | " | (+)/- |
| CAMA-1 | " | +/- |
| BT-20 | " | - |
| MDA-MB-231 | " | - |
| MaTu | " | - |
| HMEC | Normales Mammaepithel | - |
| Fibroblasten | Normales Mammagewebe | - |
| Ls174T | Kolonkarzinom | +/- |
| T-24 | Blasenkarzinom | - |
| HeLa | Zervixkarzinom | +/- |
| A-204 | Rhabdomyosarkom | +/- |
| HL-60 | Akute myeloische Leukämie | + |
| KG-1 | " | +/- |
| Molt-4 | T-Zell-Leukämie | - |

Anmerkung: Das Reaktionsmuster des Antikörpers A78-G/A7 mit den untersuchten Zellinien entspricht der Expression des TF-Antigens auf diesen Zellen

### 5.2 Ergebnisse immunhistologischer Studien an einer Reihe von menschlichen Geweben

### (Tab.4). Methodik: Paraffin-(P) oder Gefrierschnitte (G); Nachweissystem ABC

### Elite-Kit (Vector Laboratories, Burlingame, USA).

**Tabelle 4:**

| Immunhistologie mit dem Antikörper A78-G/A7 (Spezifität: Thomsen-Friedenreich-Antigen) | | | |
|---|---|---|---|
| Gewebetyp | Zahl d. Pat. | positv | % positiv |
| Mammakarzinom | | | |
| Primärtumor | 46 | 40 | 87 |
| Metastase | 23 | 21 | 91 |
| | | | |
| Normales Mammaepithel | | | |
| Randbereich | 21 | 4 | 19¹ |
| | | | |
| Kolonkarzinom | | | |
| Primärtumor | 22 | 13 | 59 |
| Metastase | 7 | 6 | 86 |
| | | | |
| Normales Kolonepithel | | | |
| Randbereich | 25 | 0 | 0 |

| | | | |
|---|---|---|---|
| ¹ Überwiegend Membran und Milchfett-Tröpfchen | | | |

## Patentansprüche

1. Verfahren zur Herstellung eines DNA-Konstrukts, bestehend aus einer DNA, welche die variable Region eines monoklonalen anti-idiotypischen Antikörpers (Ab2) gegen einen monoklonalen anti-Kohlenhydrat-Antikörper (Ab2) kodiert, **dadurch gekennzeichnet, dass** man nach an sich bekannten Verfahren monoklonale Antikörper (Ab1) gegen Antigene, die chemisch Kohlenhydrate sind, und nachfolgend anti-idiotypische Antikörper (Ab2) vom Typ β, die das Kohlenhydrat-Antigen immunologisch imitieren, herstellt und in eine diesen oder geeigneten Peptiden dieser Ab2 entsprechende cDNA nach an sich bekannten Verfahren erzeugt.

2. Verwendung des DNA-Konstrukts nach Anspruch1 zur Herstellung eines Mittels zur Behandlung von Tumorerkrankungen, bei denen tumorassoziierte Kohlenhydrat-Antigene vorhanden sind.

3. Verwendung nach Anspruch 2, **dadurch gekennzeichnet, dass** als tumorassoziiertes Antigen das Thomsen-Friedenreich-Antigen vorhanden ist.

4. Verwendung nach Anspruch 2 zur Herstellung eines Mittels zur Immunisierung gegen infektionserreger, bei denen das Zielantigen ein Kohlenhydrat ist.

## Claims

1. Method for the production of a DNA construct comprising a DNA coding the variable region of a monoclonal anti-idiotype antibody (Ab2) against a monoclonal anti-carbohydrate antibody (Ab2), wherein monoclonal antibodies (Ab1) against antigens, which chemically are carbohydrates, and subsequently type β anti-idiotype antibodies (Ab2), which immunologically imitate the carbohydrate antigen, can be produced and generated into a cDNA corresponding to these or suitable peptides of this Ab2 by methods known per se.

2. Use of the DNA construct according to Claim 1 for the production of an agent for treatment of tumour diseases, in which tumour-associated carbohydrate antigens exist.

3. Use according to Claim 2, wherein the Thomsen-Friedenreich antigen exists as a tumour-associated antigen.

4. Use according to Claim 2 for the production of an agent for immunisation against infection pathogens in which the target antigen is a carbohydrate.

## Revendications

1. Procédé de préparation d'une structure ADN, composée d'un ADN qui code la région variable d'un anticorps monoclonal anti-idiotypique (Ab2) contre un anticorps monoclonal anti-glucide (Ab2), **se caractérisant par le fait que** l'on prépare selon des procédés connus des anticorps monoclonaux (Ab1 ) contre des antigènes qui sont des glucides chimiques et ensuite des anticorps anti-idiotypiques (Ab2) du type β qui imitent au niveau immunologique l'antigène du glucide, et que l'on produit des cADN correspondant dans l'un de ces peptides ou des peptides appropriés de cet Ab2 selon des procédés connus.

2. Emploi de la structure ADN selon la revendication 1 pour la préparation d'un produit destiné au traitement de tumeurs qui présentent des antigènes du glucide associés à la tumeur.

3. Emploi selon la revendication 2, **se caractérisant par le fait que** l'antigène Thomsen-Friedenreich est l'antigène associé à la tumeur.

4. Emploi selon la revendication 2 pour la fabrication d'un produit destiné à l'immunisation contre des agents infectieux dont l'antigène cible est un glucide.
